# EUROPEAN PATENT SPECIFICATION

(11) **EP 0 550 656 B1**
(45) Date of publication and mention of the grant of the patent: **16.04.1997**
(21) Application number: 91918416.8
(22) Date of filing: 25.09.1991
(51) Int. Cl.: A61K 7/06

(54) **IMPROVED SHAMPOO COMPOSITIONS**
VERBESSERTE SHAMPOOZUSAMMENSETZUNGEN
COMPOSITIONS DE SHAMPOOING AMELIOREES

(30) Priority: 28.09.1990 US 590390; 15.02.1991 US 656935; 06.09.1991 US 755910
(43) Date of publication of application: 14.07.1993
(73) Proprietor: THE PROCTER & GAMBLE COMPANY, Cincinnati, Ohio 45202 (US)
(72) Inventor: BROCK, Earl David, Cincinnati, OH 45069 (US); LARRABEE, Antoinette Louise, Cincinnati, OH 45224 (US)
(74) Representative: Brooks, Maxim Courtney
(86) International application number: US9107024
(87) International publication number: WO9205764

(56) References cited:
- EP-A- 0 285 768
- EP-A- 0 422 508
- WO-A-83/04412

## Description

The present invention relates to improved shampoo compositions which can comprise various "performance" ingredients and a polyhydroxy fatty acid amide-type surfactant which has now been discovered to be ideally suited for use in such shampoos.

Acceptable shampoo compositions should cleanse the hair and scalp and be safe to the user, especially with regard to eye irritation. However, the modern shampoo user has come to expect more from shampooing than simple cleanliness and safety. Attention has been given to the proper selection of surfactants so that rich, creamy lathers are secured. Thickeners, colors, pearlescent agents and fragrances have been added so that shampoos have taken on more of the aspects of cosmetics. The first really effective "performance" shampoos were introduced some two decades ago when the pyridinethione antidandruff shampoos were developed. The 1980's saw the introduction of truly efficacious shampoo-plus-conditioner products into the market. Indeed, mixed shampoo-conditioner-antidandruff products have quite recently been commercialized.

Since shampooing is practiced worldwide and since the desires of the users for clean, wholesome-appearing, manageable hair appear to be universal, it is not surprising that shampoo formulators and manufacturers have directed considerable resources to the discovery and development of new ingredients, especially for performance shampoos, such as new conditioning agents, improved antidandruff agents, better styling agents, and the like. The patent literature, cosmetic journals and formularies are replete with reports of such new agents.

The present invention represents a departure from most current lines of research into the formulation of shampoos, especially performance shampoos. Simply stated, the present invention is based on the discovery that a known class of surfactants, the polyhydroxy fatty acid amides, not only provide the rich lather properties so desirable in shampoos, but also exhibit desirable mildness to the eye and can accentuate the benefits of various performance agents.

A variety of polyhydroxy fatty acid amides have been described in the art. N-acyl, N-methyl glucamines, for example, are disclosed by J. W. Goodby, M. A. Marcus, E. Chin, and P. L. Finn in "The Thermotropic Liquid-Crystalline Properties of Some Straight Chain Carbohydrate Amphiphiles," Liquid Crystals, 1988, Volume 3, No. 11, pp 1569-1581, and by A. Muller-Fahrnow, V. Zabel, M. Steifa, and R. Hilgenfeld in "Molecular and Crystal Structure of a Nonionic Detergent: Nonanoyl-N-methylglucamide," J. Chem. Soc. Chem. Commun., 1986, pp 1573-1574. The use of N-alkyl polyhydroxyamide surfactants has been of substantial interest recently for use in biochemistry, for example in the dissociation of biological membranes. See, for example, the journal article "N-D-Gluco-N-methyl-alkanamide Compounds, a New Class of Non-Ionic Detergents For Membrane Biochemistry," Biochem. J. (1982), Vol. 207, pp 363-366, by J. E. K. Hildreth.

The use of N-alkyl glucamides in detergent compositions has also been discussed. US-A-2,965,576, issued December 20, 1960 to E. R. Wilson, and GB-A-809,060, published February 18, 1959, assigned to Thomas Hedley & Co., Ltd. relate to detergent compositions containing anionic surfactants and certain amide surfactants, which can include N-methyl glucamide, added as a low temperature suds enhancing agent. These compounds include an N-acyl radical of a higher straight chain fatty acid having 10-14 carbon atoms. These compositions may also contain auxiliary materials such as alkali metal phosphates, alkali metal silicates, sulfates, and carbonates. It is also generally indicated that additional constituents to impart desirable properties to the composition can also be included in the compositions, such as fluorescent dyes, bleaching agents, perfumes, etc.

US-A-2,703,798, issued March 8, 1955 to A. M. Schwartz, relates to aqueous detergent compositions containing the condensation reaction product of N-alkyl glucamine and an aliphatic ester of a fatty acid. The product of this reaction is said to be useable in aqueous detergent compositions without further purification. It is also known to prepare a sulfuric ester of acylated glucamine as disclosed in US-A-2,717,894, issued September 13, 1955, to A. M. Schwartz.

WO-A-83/04412, published December 22, 1983, by J. Hildreth, relates to amphiphilic compounds containing polyhydroxyl aliphatic groups said to be useful for a variety of purposes including use as surfactants in cosmetics, drugs, shampoos, lotions, and eye ointments, as emulsifiers and dispensing agents for medicines, and in biochemistry for solubilizing membranes, whole cells, or other tissue samples, and for preparation of liposomes. Included in this disclosure are compounds of the formula R'CON(R)CH₂R'' and R''CON(R)R' wherein R is hydrogen or an organic grouping, R' is an aliphatic hydrocarbon group of at least three carbon atoms, and R'' is the residue of an aldose.

EP-A-0 285 768, published October 12, 1988, H. Kelkenberg, et al., relates to the use of N-polyhydroxy alkyl fatty acid amides as thickening agents in aqueous detergent systems. Included are amides of the formula R₁C(O)N(X)R₂ wherein R₁ is a C₁-C₁₇ (preferably C₇-C₁₇) alkyl, R₂ is hydrogen, a C₁-C₁₈ (preferably C₁-C₆) alkyl, or an alkylene oxide, and X is a polyhydroxy alkyl having four to seven carbon atoms, e.g., N-methyl, coconut fatty acid glucamide. The thickening properties of the amides are indicated as being of particular use in liquid surfactant systems containing paraffin sulfonate, although the aqueous surfactant systems can contain other anionic surfactants, such as alkylaryl sulfonates, olefin sulfonate, sulfosuccinic acid half ester salts, and fatty alcohol ether sulfonates, and nonionic surfactants such as fatty alcohol polyglycol ether, alkylphenol polyglycol ether, fatty acid polyglycol ester, polypropylene oxide-polyethylene oxide mixed polymers, etc. Paraffin sulfonate/N-methyl coconut fatty acid glucamide/nonionic surfactant shampoo formulations are exemplified. In addition to thickening attributes, the N-polyhydroxy alkyl fatty acid amides are said to have superior skin tolerance attributes.

US-A-2,982,737, issued May 2, 1961, to Boettner, et al., relates to detergent bars containing urea, sodium lauryl sulfate anionic surfactant, and an N-alkylglucamide nonionic surfactant which is selected from N-methyl,N-sorbityl lauramide and N-methyl, N-sorbityl myristic acid amide.

Other glucamide surfactants are disclosed, for example, in DT-A-2,226,872, published December 20, 1973, H. W. Eckert, et al., which relates to washing compositions comprising one or more surfactants and builder salts selected from polymeric phosphates, sequestering agents, and washing alkalis, improved by the addition of an N-acylpolyhydroxy-alkyl-amine of the formula R₁C(O)N(R₂)CH₂(CHOH)ₙCH₂OH, wherein R₁ is a C₁-C₃ alkyl, R₂ is a C₁₀-C₂₂ alkyl, and n is 3 or 4. The N-acylpolyhydroxyalkyl-amine is added as a soil suspending agent.

US-A-3,654,166, issued April 4, 1972, to H. W. Eckert, et al., relates to detergent compositions comprising at least one surfactant selected from the group of anionic, zwitterionic, and nonionic surfactants and, as a textile softener, an N-acyl, N-alkyl polyhydroxylalkyl compound of the formula R₁N(Z)C(O)R₂ wherein R₁ is a C₁₀-C₂₂ alkyl, R₂ is a C₇-C₂₁ alkyl, R₁ and R₂ total from 23 to 39 carbon atoms, and Z is a polyhydroxyalkyl which can be -CH₂(CHOH)ₘCH₂OH where m is 3 or 4.

U-S-A-4,021,539, issued May 3, 1977, to H. Möller, et al., relates to skin treating cosmetic compositions containing N-polyhydroxylalkyl-amines which include compounds of the formula R₁N(R)CH(CHOH)ₘR₂ wherein R₁ is H, lower alkyl, hydroxy-lower alkyl, or aminoalkyl, as well as heterocyclic aminoalkyl, R is the same as R₁ but both cannot be H, and R₂ is CH₂OH or COOH.

FR-A-1,360,018, April 26, 1963, assigned to Commercial Solvents Corporation, relates to solutions of formaldehyde stabilized against polymerization with the addition of amides of the formula RC(O)N(R₁)G wherein R is a carboxylic acid functionality having at least seven carbon atoms, R₁ is hydrogen or a lower alkyl group, and G is a glycitol radical with at least 5 carbon atoms.

DE-A-1,261,861, February 29, 1968, A. Heins, relates to glucamine derivatives useful as wetting and dispersing agents of the formula N(R)(R₁)(R₂) wherein R is a sugar residue of glucamine, R₁ is a C₁₀-C₂₀ alkyl radical, and R₂ is a C₁-C₅ acyl radical.

GB-A-745,036, published February 15, 1956, assigned to Atlas Powder Company, relates to heterocyclic amides and carboxylic esters thereof that are said to be useful as chemical intermediates, emulsifiers, wetting and dispersing agents, detergents, textile softeners, etc. The compounds are expressed by the formula N(R)(R₁)C(O)R₂ wherein R is the residue of an anhydrized hexane pentol or a carboxylic acid ester thereof, R₁ is a monovalent hydrocarbon radical, and -C(O)R₂ is the acyl radical of a carboxylic acid having from 2 to 25 carbon atoms.

US-A-3,312,627, issued April 4, 1967 to D. T. Hooker, discloses solid toilet bars that are substantially free of anionic detergents and alkaline builder materials, and which contain lithium soap of certain fatty acids, a nonionic surfactant selected from certain propylene oxide-ethylenediamine-ethylene oxide condensates, propylene oxide-propylene glycol-ethylene oxide condensates, and polymerized ethylene glycol, and also contain a nonionic lathering component which can include polyhydroxyamide of the formula RC(O)NR¹(R²) wherein RC(O) contains from about 10 to about 14 carbon atoms, and R¹ and R² each are H or C₁-C₆ alkyl groups, said alkyl groups containing a total number of carbon atoms of from 2 to about 7 and a total number of substituent hydroxyl groups of from 2 to about 6. A substantially similar disclosure is found in US-A-3,312,626, also issued April 4, 1967 to D. T. Hooker.

The present invention encompasses shampoo compositions comprising a silicone-containinghair conditioning agent at a level of from 0,05-10% by weight; said compositions optionally comprising one or more adjunct surfactants (especially, anionic surfactants), optional thickening agents and fluid carrier, said composition comprising at least 1% by weight of a polyhydroxy fatty acid amide surfactant.

Preferred compositions herein are those wherein said polyhydroxy fatty acid amide surfactant is of the formula R²C(O)NR¹CH₂(CH₂OH)₄CH₂OH, wherein R² is C₁₁-C₁₇ alkyl and R¹ is methyl.

Preferred conditioning compositions according to this invention are those wherein the hair conditioning agent is a silicone. The silicone conditioning agent is most preferably used in conjunction with a thickening agent, especially ethylene glycol distearate.

The present invention also encompasses a method for enhancing the deposition of a silicone-containing hair conditioning agent at a level of from 0,05-10% by weight; onto hair from a shampoo vehicle comprising including in said shampoo vehicle at least 1% by weight of a polyhydroxy fatty acid amide, and shampooing the hair with the resulting composition.

All percentages and ratios herein are by weight, unless otherwise specified.

The present invention employs various ingredients, many of which can be illustrated by reference to the extensive cosmetics literature, and it is to be understood that the manufacture of such ingredients *per se* forms no part of this invention. However, the following nonlimiting exemplifications will assist the formulator in obtaining the appropriate ingredients for formulating the compositions herein.

### Polyhydroxy Fatty Acid Amides

The compositions herein comprise at least 1%, typically from 5% to 50%, preferably from 7% to 20% by weight, of the polyhydroxy fatty acid amide surfactant described below.

The polyhydroxy fatty acid amide surfactant component of the present invention comprises compounds of the structural formula: 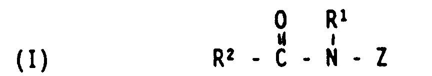 wherein: R¹ is H, C₁-C₄ hydrocarbyl, 2-hydroxy ethyl, 2-hydroxy propyl, or a mixture thereof, preferably C₁-C₄ alkyl, more preferably C₁ or C₂ alkyl, most preferably C₁ alkyl (i.e., methyl); and R² is a C₅-C₃₁ hydrocarbyl moiety, preferably straight chain C₇-C₁₉ alkyl or alkenyl, more preferably straight chain C₉-C₁₇ alkyl or alkenyl, most preferably straight chain C₁₁-C₁₅ alkyl or alkenyl, or mixture thereof; and Z is a polyhydroxyhydrocarbyl moiety having a linear hydrocarbyl chain with at least 3 hydroxyls directly connected to the chain, or an alkoxylated derivative (preferably ethoxylated or propoxylated) thereof. Z preferably will be derived from a reducing sugar in a reductive amination reaction; more preferably Z is a glycityl moiety. Suitable reducing sugars include glucose, fructose, maltose, lactose, galactose, mannose, and xylose. As raw materials, high dextrose corn syrup, high fructose corn syrup, and high maltose corn syrup can be utilized as well as the individual sugars listed above. These corn syrups may yield a mix of sugar components for Z. It should be understood that it is by no means intended to exclude other suitable raw materials. Z preferably will be selected from the group consisting of -CH₂-(CHOH)ₙ-CH₂OH, -CH(CH₂OH)-(CHOH)ₙ₋₁-CH₂OH, -CH₂-(CHOH)₂(CHOR')(CHOH)-CH₂OH, where n is an integer from 3 to 5, inclusive, and R' is H or a cyclic or aliphatic monosaccharide, and alkoxylated derivatives thereof. Most preferred are glycityls wherein n is 4, particularly -CH₂-(CHOH)₄-CH₂OH.

In Formula (I), R¹ can be, for example, N-methyl, N-ethyl, N-propyl, N-isopropyl, N-butyl, N-2-hydroxy ethyl, or N-2-hydroxy propyl.

R²-CO-N< can be, for example, cocamide, stearamide, oleamide, lauramide, myristamide, capricamide, palmitamide, tallowamide, etc.

Z can be 1-deoxyglucityl, 2-deoxyfructityl, 1-deoxymaltityl, 1-deoxylactityl, 1-deoxygalactityl, 1-deoxymannityl, 1-deoxymaltotriotityl, etc.

Methods for making polyhydroxy fatty acid amides are known in the art. In general, they can be made by reacting an alkyl amine with a reducing sugar in a reductive amination reaction to form a corresponding N-alkyl polyhydroxyamine, and then reacting the N-alkyl polyhydroxyamine with a fatty aliphatic ester or triglyceride in a condensation/amidation step to form the N-alkyl, N-polyhydroxy fatty acid amide product. Processes for making compositions containing polyhydroxy fatty acid amides are disclosed, for example, in GB-A-809,060, published February 18, 1959, by Thomas Hedley & Co., Ltd., US-A-2,965,576, issued December 20, 1960 to E. R. Wilson, and US-A-2,703,798, Anthony M. Schwartz, issued March 8, 1955, and US-A-1,985,424, issued December 25, 1934 to Piggot.

In one process for producing N-alkyl or N-hydroxyalkyl, N-deoxyglycityl fatty acid amides wherein the glycityl component is derived from glucose and the N-alkyl or N-hydroxyalkyl functionality is N-methyl, N-ethyl, N-propyl, N-butyl, N-hydroxyethyl, or N-hydroxypropyl, the product is made by reacting N-alkyl- or N-hydroxyalkyl-glucamine with a fatty ester selected from fatty methyl esters, fatty ethyl esters, and fatty triglycerides in the presence of a catalyst selected from the group consisting of trilithium phosphate, trisodium phosphate, tripotassium phosphate, tetrasodium pyrophosphate, pentapotassium tripolyphosphate, lithium hydroxide, sodium hydroxide, potassium hydroxide, calcium hydroxide, lithium carbonate, sodium carbonate, potassium carbonate, disodium tartrate, dipotassium tartrate, sodium potassium tartrate, trisodium citrate, tripotassium citrate, sodium basic silicates, potassium basic silicates sodium basic aluminosilicates, and potassium basic aluminosilicates, and mixtures thereof. The amount of catalyst is preferably from about 0.5 mole % to about 50 mole %, more preferably from about 2.0 mole % to about 10 mole %, on an N-alkyl or N-hydroxyalkyl-glucamine molar basis. The reaction is preferably carried out at from about 138°C to about 170°C for typically from about 20 to about 90 minutes. When triglycerides are utilized in the reaction mixture as the fatty ester source, the reaction is also preferably carried out using from 1 to 10 weight % of a phase transfer agent, calculated on a weight percent basis of total reaction mixture, selected from saturated fatty alcohol polyethoxylates, alkylpolyglucosides, linear glucamide surfactant, and mixtures thereof.

Preferably, this process is carried out as follows:
(a) preheating the fatty ester to about 138°C to about 170°C;
(b) adding the N-alkyl or N-hydroxyalkyl glucamine to the heated fatty acid ester and mixing to the extent needed to form a two-phase liquid/liquid mixture;
(c) mixing the catalyst Into the reaction mixture; and
(d) stirring for the specified reaction time.

Also preferably, from 2% to 20% by weight of preformed linear N-alkyl or N-hydroxyalkyl, N-linear glucosyl fatty acid amide product is added to the reaction mixture, by weight of the reactants, as the phase transfer agent if the fatty ester is a triglyceride. This seeds the reaction, thereby increasing reaction rate.

The polyhydroxy "fatty acid" amide materials used herein also offer the advantages to the shampoo formulator that they can be prepared wholly or primarily from natural, renewable, nonpetrochemical feedstocks and are degradable. They also exhibit low toxicity to aquatic life.

It should be recognized that along with the polyhydroxy fatty acid amides of Formula (I), the processes used to produce them will also typically produce quantities of nonvolatile by-product such as esteramides and cyclic polyhydroxy fatty acid amide. The level of these by-products will vary depending upon the particular reactants and process conditions. Preferably, the polyhydroxy fatty acid amide incorporated into the shampoo compositions herein will be provided in a form such that the polyhydroxy fatty acid amide contains less than 10%, preferably less than 4% by weight, of cyclic polyhydroxy fatty acid amide. The preferred processes described above are advantageous in that they can yield rather low levels of by-products, Including such cyclic amide by-product.

### Hair Conditioning Agents

Various materials have been taught in the art for use as agents which condition the hair. In general, such conditioning agents are designed to enhance the fullness (or "body"), manageability or "combability", softness, luster and overall attractive appearance and handling properties of the hair. It is to be understood that any such conditioning agents can be employed herein, according to the desires of the formulator, but that silicone hair conditioning agents are especially preferred.

Silicone fluids are a suitable nonvolatile silicone that may be used in the present composition. US-A-3,742,855 can be referred to for details of various silicones used in performance shampoos.

The nonvolatile silicone fluid may be either an insoluble polyalkyl siloxane, a polyaryl siloxane, a polyalkylaryl siloxane or a polyether siloxane copolymer. Mixtures of these fluids may also be used and are preferred in certain executions. The dispersed silicone particles should also be insoluble in the shampoo matrix. This is the meaning of "insoluble" as used hereinbefore and hereinafter.

The essentially nonvolatile polyalkyl siloxane fluids that may be used include, for example, polydimethyl siloxanes with viscosities ranging from 5 x mm²s⁻¹ to 600,000 x mm²s⁻¹ (5 to 600,000 centistokes) at 25°C. These siloxanes are available, for example, from the General Electric Company as the Viscasil (RTM) series and from Dow Corning as the Dow Corning 200 (RTM) series. The viscosity can be measured by means of a glass capillary viscometer as set forth in Dow Corning Corporate Test Method CTM0004, July 20, 1970. Preferably the viscosity ranges from about 350 x mm²s⁻¹ (350 centistokes) to about 100,000 x mm²s⁻¹ (100,000 centistokes).

The essentially nonvolatile polyalkylaryl siloxane fluids that may be used include, for example, polymethylphenylsiloxanes having viscosities of 15 x mm²s⁻¹ to 30,000 x mm²s⁻¹ (15 to 30,000 centistokes) at 25°C. These siloxanes are available, for example, from the General Electric Company as SF 1075 methyl phenyl fluid or from Dow Corning as 556 Cosmetic Grade Fluid.

The essentially nonvolatile polyether siloxane copolymer that may be used is, for example, a polypropylene oxide modified dimethylpolysiloxane (e.g., Dow Corning DC-1248 (RTM)) although ethylene oxide may also be used.

References disclosing suitable silicone fluids include US-A-2,826,551 to Geen; US-A-3,964,500, June 22, 1976 to Drakoff; US-A-4,364,837 to Pader and GB-A-849,433 to Woolston. *Silicon Compounds* distributed by Petrarch Systems, Inc., 1984 provides a very good listing of suitable silicone materials.

Another silicone material useful in the present compositions to provide good dry combing is a silicone gum. Silicone gums are described by Petrarch and others including US-A-4,152,416 May 1, 1979 to Spitzer et al, and Noll, Walter, *Chemistry and Technology of Silicones*, New York: Academic Press 1968. Also describing silicone gums are General Electric Silicone Rubber Product Data Sheets SE 30, SE 33, SE 54 and SE76. "Silicone gum" materials denote high molecular weight polydiorganosiloxanes having a mass molecular weight of from about 200,000 to about 1,000,000. Specific examples include polydimethylsiloxane, (polydimethylsiloxane) (methylvinylsiloxane) copolymer, poly(dimethylsiloxane) (diphenyl) (methylvinylsiloxane) copolymer and mixtures thereof.

Silicone hair conditioning agents are used in composition of the present type at levels from at least 0.05% to 10% (wt.), preferably from 0.2% to 7%, even more preferably from 0.5% to 5%, and in some compositions at preferred levels of from 1% to 2% by weight. Silicones are typically used in combination with suspending agents, as disclosed more fully hereinafter.

Other hair conditioning agents useful herein include various quaternary ammonium and amine compounds well-known to formulators of shampoos and hair conditioners. Nonlimiting examples of such materials include tri long chain alkyl mono short chain alkyl quaternary ammonium salts and tri long chain amines. By "long" is meant having from about 8 to about 22 carbon atoms while "short" includes alkyls having from about 1 to about 4 carbon atoms. A preferred material is tricetyl methyl ammonium chloride. Other halides such as bromide and iodide or organic groups such as methyl sulfate may be used to form the salt. Other specific compounds include tri C₈₋₁₀ methyl ammonium chloride, tri(isodecyl)amine and tri C₁₃ amine. Quaternary compounds or amine can be used at a level of from 0.1% to 4%, preferably from 0.25% to 2% by weight.

### Suspending Agents

As noted hereinabove, various performance ingredients used in the present shampoo compositions are advantageously used in combination with a thickening agent. Indeed, the suspension of materials such as silicone particles or drops, and the like, in the shampoos in a substantially uniform suspension assists in providing the desirable performance attributes associated with these ingredients. Said suspending agents are typically used to bring the viscosity of the compositions to from about 8,000 mPa.s to about 20,000 mPa.s (8,000 cp to about 20,000 cp) as measured with a Wells-Brookfield viscometer, Model RVT DV-CP-2, DV-11, Model Cone CP-52 using 1/2 ml at 1 rpm at 26.7°C for 1 minute. Of course, this can be varied, according to the desires of the formulator.

Xanthan gum is a suspending agent which can be used in the present compositions to suspend, for example, the silicone fluid. This biosynthetic gum material is commercially available and is a heteropolysaccharide with a molecular weight of greater than 1 million. It contains D-glucose, D-mannose and D-glucuronate in the molar ratio of 2.8:2.0:2.0. The polysaccharide is partially acetylated with 4.7% acetyl. This information and other is found in Whistler, Roy L. Editor *Industrial Gums - Polysaccharides and Their Derivatives* New York: Academic Press, 1973. Kelco, a Division of Merck & Co., Inc. offers xanthan gum as KELTROL (RTM). The gum is typically used at a level of from 0.4% to 3%, preferably from 0.6% to by weight 1.2% in the compositions of the present invention.

Another suspending agent useful in the present compositions is any of several long chain acyl derivatives materials or mixtures of such materials. Included are ethylene glycol esters of fatty acids having from about 16 to about 22 carbon atoms. Preferred are the ethylene glycol stearates, both mono and distearate ("EGDS") but particularly the distearate containing less than about 7% of the mono stearate. Other suspending agents found useful are alkanol amides of fatty acids, having from about 16 to about 22 carbon atoms, preferably about 16 to 18 carbon atoms. Preferred alkanol amides are stearic monoethanolamide, stearic diethanolamide, stearic monoisopropanolamide and stearic monoethanolamide stearate. Still other suitable nonacyl derivative suspending agents are alkyl(C₁₆₋₂₂)dimethyl amine oxides such as stearyl dimethyl amine oxide.

The acyl or nonacyl suspending agent or mixtures of agents is typically used at a level of from 0.4% to 5%, preferably from 0.5% to 3.0% by weight. The suspending agent, especially EGDS, serves to assist in suspending silicone materials and to give a pearlescent appearance to the product.

### Optional Adjunct Surfactants

The shampoos herein will optionally, but preferably, contain surfactants in addition to the polyhydroxy fatty acid amide. A wide variety of surfactants have been suggested in the literature for use in shampoos, and any such surfactants can be used herein, according to the desires of the formulator. Reference can be made to standard texts, such as the "McCutcheon's Index" for listings, so an exhaustive listing is not provided here. However, to assist the formulator and not by way of limitation, the following suitable adjunct surfactants can be mentioned: soaps, including sodium potassium and triethanolammonium salts of C₁₂-C₂₀ fatty acids; sodium potassium and triethanolammonium salts of C₁₂-C₂₀ alkyl sulfates, alkyl ethoxy sulfates, and alkyl phenol ethylene oxide ether sulfates; nonionic surfactants, especially coconut alcohol ethoxylates; alkyl betaines and the like.

Especially preferred adjunct surfactants herein are the C₁₂-C₁₈ ethoxylated alkylether sulfates with at least 2, preferably 3 or more ethoxy units. Coconutalkyl (EO)₃ sulfate, ammonium salt, is especially preferred.

The adjunct surfactant is typically used at levels from 1.0% to 20%, preferably from 3% to 9% by weight of the shampoo compositions.

Preferred weight ratios of the adjunct surfactant to polyhydroxy fatty acid amide range from 5:1 to 1:2.

### Fluid Carrier

The compositions herein can be in the form of liquids, thickened liquids or gels. Water is generally used as the carrier fluid and will typically be present at levels from 20% to 95% by weight

### Optional Ingredients

The shampoos herein can contain a variety of nonessential optional components suitable for rendering such compositions more acceptable. Such conventional optional ingredients are well known to those skilled in the art, e.g., preservatives such as benzyl alcohol, methyl paraben, propyl paraben and imidazolidinyl urea; other cationic surfactants such as lauryl trimethyl ammonium chloride, cetyl trimethyl ammonium chloride, stearyldimethyl benzyl ammonium chloride and di(partially hydrogenated tallow) dimethylammonium chloride; thickeners and viscosity modifiers such as a diethanolamide of a long chain fatty acid (e.g., Cocamide MEA), amine oxides, block polymers of ethylene oxide and propylene oxide such as Pluronic F88 (RTM) offered by BASF Wyandotte, fatty alcohols such as cetearyl alcohol, sodium chloride, sodium sulfate, polyvinyl alcohol, and ethyl alcohol; pH adjusting agents such as monosodium phosphate and disodium phosphate citric acid, succinic acid, phosphoric acid, sodium hydroxide, sodium carbonate, etc.; perfumes; dyes; and, sequestering agents such as disodium ethylenediamine tetraacetate. Such agents generally are used individually at a level of from 0.01% to 10%, preferably from 0.5% to 5.0% by weight of the composition.

Another optional ingredient and one preferred for use in certain of the compositions of this invention, is a volatile silicone or a water-insoluble hydrocarbon. These agents are disclosed in US-A-4,472,375, September 18, 1984 to R. E. Bolich, Jr.. These agents help disperse the higher molecular weight nonvolatile silicones in the product when such silicones are used. These agents are used at levels of from 0.1% to 5% by weight.

The pH of the present compositions can be in the range of from 4 to about 10.

The following Examples further illustrate the practice of this invention, but are not intended to be limiting thereof.

### EXAMPLE I

A typical synthesis is as follows, and exemplifies a process for making a N-methyl-N-glucosyl lauramide surfactant for use in the present shampoo compositions. Although a skilled chemist can vary apparatus configuration, one suitable apparatus for use herein comprises a three-liter four-necked flask fitted with a motor-driven paddle stirrer and a thermometer of length sufficient to contact the reaction medium. The other two necks of the flask are fitted with a nitrogen sweep and a wide-bore side-arm (caution: a wide-bore side-arm is important in case of very rapid methanol evolution) to which is connected an efficient collecting condenser and vacuum outlet. The latter is connected to a nitrogen bleed and vacuum gauge, then to an aspirator and a trap. A 500 watt heating mantle with a variable transformer temperature controller ("Variac") used to heat the reaction is so placed on a lab-jack that it may be readily raised or lowered to further control temperature of the reaction.

N-methylglucamine (195 g., 1.0 mole, Aldrich, M4700-0) and methyl laurate (Procter & Gamble CE 1270, 220.9 g., 1.0 mole) are placed in a flask. The solid/liquid mixture is heated with stirring under a nitrogen sweep to form a melt (approximately 25 minutes). When the melt temperature reaches 145°C, catalyst (anhydrous powdered sodium carbonate, 10.5 g., 0.1 mole, J. T. Baker) is added. The nitrogen sweep iss shut off and the aspirator and nitrogen bleed are adjusted to give 16.95 kNm⁻² (5 inches (5/31 atm.) Hg) vacuum. From this point on, the reaction temperature is held at 150°C by adjusting the variac and/or by raising or lowering the mantle.

Within 7 minutes, first methanol bubbles are sighted at the meniscus of the reaction mixture. A vigorous reaction soon follows. Methanol is distilled over until its rate subsides. Then adjust vacuum to give about 33.9 kNm⁻² (10 inches Hg. (10/31 atm.)) vacuum. The vacuum is increased approximately as follows (in inches Hg. at minutes): 10 at 3, 20 at 7, 25 at 10. 11 minutes from the onset of methanol evolution, heating and stirring are discontinued coincident with some foaming. Analysis by TLC shows that at this point the process is complete. The reaction product is cooled and solidifies. The alkyl N-methyl glucamide product comprises C₁₁H₂₃C(O)N(CH₃)CH₂(CHOH)₄CH₂OH and is typical of the polyhydroxy fatty acid amide surfactants used herein.

In similar fashion, methyl esters of fatty acid mixtures (especially C₁₂-C₁₈ acids) derived from coconut oil (preferred), palm oil and soybean oil, respectively, are reacted in the foregoing manner to provide polyhydroxy fatty acid amide surfactants (mixed alkyl N-methyl glucamides) for use herein.

### EXAMPLE II

A conditioning shampoo is as follows. The method of manufacture noted is generally acceptable for preparing the other shampoo compositions herein. However, shampoo formulators will appreciate that variations in manufacturing methods are possible.

| Component | Weight % |
|---|---|
| Coconutalkyl (EO)₃ sulfate (NH₄ salt) | 13.5 |
| Coconutalkyl N-methyl glucose amide¹ | 3.5 |
| Ethylene glycol distearate | 3.0 |
| Dimethicone² | 1.0 |
| Ammonium chloride | 3.00 |
| Tricetyl methyl ammonium chloride | 0.50 |
| Cetyl alcohol | 0.42 |
| Stearyl alcohol | 0.18 |
| Citric acid | 0.16 |
| Perfume | 0.65 |
| Preservative (GLYDANT (RTM)) | 5 ppm |
| Water (double reverse osmosis) | Balance |

| | |
|---|---|
| ¹Prepared per Example I | |
| ²1:1 (wt.) mixture silicone gum:silicone fluid | |

In a typical manufacturing procedure a premix containing the silicone hair conditioning agent is prepared. The premix comprises alkylethoxylated sulfate heated to 170°F (77°C) ±10° to which is added a portion of the stearyl alcohol at the same temperature, to which is then added a portion of the cetyl alcohol at the same temperature, with mixing for a minimum of about 20 minutes. The temperature is then increased to 180°F (82°C) before silicone addition. The silicone is then added at 180°F (82°C) ±5° and mixed for 60 minutes.

A mix is prepared using water at 170°F (77°C) ±10° to which is added the fatty acid polyhydroxy amide surfactant at the same temperature, to which is subsequently added a portion of the cetyl alcohol and a portion of the stearyl alcohol, respectively, at the same temperature, followed by addition of the ethylene glycol distearate at that temperature, followed by the addition of the tricetyl methyl ammonium chloride, at which time the system is mixed for a minimum of about 11 minutes ± 3 minutes, typically over a range from about 8 minutes to about 35 minutes. The silicone premix is added at 170°F (77°C) ±10°. The preservative is then added at the same temperature and mixing is continued for 5-30 minutes.

The balance of the ingredients are then added, generally at about 80°F (27.5°C) to provide the final product.

### EXAMPLE III

An alternate method for preparing the polyhydroxy fatty acid amides used herein is as follows. A reaction mixture consisting of 84.87 g. fatty acid methyl ester (source: Procter & Gamble methyl ester CE1270), 75 g. N-methyl-D-glucamine (source: Aldrich Chemical Company M4700-0), 1.04 g. sodium methoxide (source: Aldrich Chemical Company 16,499-2) and 68.51 g. methyl alcohol is used. The reaction vessel comprises a standard reflux set-up fitted with a drying tube, condenser and stir bar. In this procedure, the N-methyl glucamine is combined with methanol with stirring under argon and heating is begun with good mixing (stir bar; reflux). After 15-20 minutes, when the solution has reached the desired temperature, the ester and sodium methoxide catalyst are added. Samples are taken periodically to monitor the course of the reaction, but it is noted that the solution is completely clear by 63.5 minutes. It is judged that the reaction is, in fact, nearly complete at that point. The reaction mixture is maintained at reflux for 4 hours. After removal of the methanol, the recovered crude product weighs 156.16 grams. After vacuum drying and purification, an overall yield of 106.92 grams purified product is recovered. However, percentage yields are not calculated on this basis, inasmuch as regular sampling throughout the course of the reaction makes an overall percentage yield value meaningless. The reaction can also be carried out at 80% and 90% reactant concentrations.

Compositions herein with improved suds qualities can be formulated using a suds-enhancing amount (typically, from 0.5% to 10%, preferably 2.5% to 4% by weight of the final composition) of an alkyliminodipropionate or "alkylamphodiacetate" (e.g., lauroamphocarboxyglycinate) (alkyl typically in the C₁₂-C₁₈ range in both instances) surfactant. Such materials are commercially available as DERIPHAT 160 (RTM) and MIRANOL H2M (RTM).

More generally, such surfactants are of the known classes of amphoteric surfactants which include alkylaminoalkanoates of the type R-NH(CH₂)ₙCOOM and alkyliminodialkanoates of the type R-N[(CH₂)ₘCOOM]₂ and mixtures thereof; wherein n and m are integers from 1 to 4, R is C₈-C₂₂ alkyl or alkenyl, and M is hydrogen, alkali metal, alkaline earth metal, ammonium or alkanolammonium.

Preferred examples of amphoteric surfactants include n-alkylamino-propionates and n-alkyliminodipropionates. Such materials are sold under the tradename DERIPHAT by Henkel and MIRATAINE by Miranol, Inc. Most preferred for use in the present compositions are N-lauryl-beta-amino propionic acid or salts thereof, and N-lauryl-beta-imino-dipropionic acid ("lauroiminodipropionate") (DERIPHAT (RTM) 160C) or salts thereof, and mixtures thereof. The "cocoamphodiacetates" are also useful herein.

The total level of anionic surfactant plus amphoteric surfactants in the present compositions is preferably from 5% to 20%, preferably from 9% to 18% by weight. The ratio of anionic surfactant to amphoteric surfactant in the present compositions is generally from 0.5:1 to 5:1. The following Example illustrates a composition of this type.

### EXAMPLE IV

| Ingredient | % Active (wt.) |
|---|---|
| Zinc pyrithione | 1.0 |
| Ammonium laureth-3 sulfate | 8.47 |
| Lauroiminodipropionate | 3.5 |
| Coconutalkyl N-methylglucamide | 8.45 |
| Ethylene glycol distearate | 3.0 |
| Silicone* | 1.0 |
| Stearyl alcohol | 0.18 |
| Cetyl alcohol | 0.42 |
| Citric acid | 0.13 |
| Glydant** (RTM) | 0.20 |
| Perfume | 0.65 |
| Color | 0.04 |
| Water | Balance |

| | |
|---|---|
| * Dimethicone - General Electric Company. | |
| ** Preservative - W. R. Grace Chemical Company. Kathon (5 ppm) (RTM) may also be used. | |
| ***The composition can also contain 0.55% of ammonium xylene sulfonate. | |

The following is not intended to limit the invention herein, but is simply to further illustrate additional aspects of the technology which may be considered by the formulator in the manufacture of a wide variety of detergent compositions using the polyhydroxy fatty acid amides.

It will be readily appreciated that the polyhydroxy fatty acid amides are, by virtue of their amide bond, subject to some instability under highly basic or highly acidic conditions. While some decomposition can be tolerated, it is preferred that these materials not be subjected to pH's above about 11, preferably 10, nor below about 3 for unduly extended periods. Final product pH (liquids) is typically 7.0-9.0.

During the manufacture of the polyhydroxy fatty acid amides it will typically be necessary to at least partially neutralize the base catalyst used to form the amide bond. While any acid can be used for this purpose, the detergent formulator will recognize that it is a simple and convenient matter to use an acid which provides an anion that is otherwise useful and desirable in the finished detergent composition. For example, citric acid can be used for purposes of neutralization and the resulting citrate ion (*ca.* 1%) be allowed to remain with a *ca.* 40% polyhydroxy fatty acid amide slurry and be pumped into the later manufacturing stages of the overall detergent-manufacturing process. The acid forms of materials such as oxydisuccinate, nitrilotriacetate, ethylenediaminetetraacetate, tartrate/succinate, and the like, can be used similarly.

The polyhydroxy fatty acid amides derived from coconut alkyl fatty acids (predominantly C₁₂-C₁₄) are more soluble than their tallow alkyl (predominantly C₁₆-C₁₈) counterparts. Accordingly, the C₁₂-C₁₄ materials are somewhat easier to formulate in liquid compositions, and are more soluble in cool-water laundering baths. However, the C₁₆-C₁₈ materials are also quite useful, especially under circumstances where warm-to-hot wash water is used. Indeed, the C₁₆-C₁₈ materials may be better detersive surfactants than their C₁₂-C₁₄ counterparts. Accordingly, the formulator may wish to balance ease-of-manufacture *vs.* performance when selecting a particular polyhydroxy fatty acid amide for use in a given formulation.

It will also be appreciated that the solubility of the polyhydroxy fatty acid amides can be increased by having points of unsaturation and/or chain branching in the fatty acid moiety. Thus, materials such as the polyhydroxy fatty acid amides derived from oleic acid and iso-stearic acid are more soluble than their n-alkyl counterparts.

Likewise, the solubility of polyhydroxy fatty acid amides prepared from disaccharides, trisaccharides, etc., will ordinarily be greater than the solubility of their monosaccharide-derived counterpart materials. This higher solubility can be of particular assistance when formulating liquid compositions. (The manufacture of a polyhydroxy fatty acid amide derived from maltose is described hereinafter.)

The polyhydroxy fatty acid amides can be manufactured not only from the purified sugars, but also from hydrolyzed starches, e.g., corn starch, potato starch, or any other convenient plant-derived starch which contains the mono-, di-, etc. saccharide desired by the formulator. This is of particular importance from the economic standpoint. Thus, "high glucose" corn syrup, "high maltose" corn syrup, etc. can conveniently and economically be used. De-lignified, hydrolyzed cellulose pulp can also provide a raw material source for the polyhydroxy fatty acid amides.

As noted above, polyhydroxy fatty acid amides derived from the higher saccharides, such as maltose, lactose, etc., are more soluble than their glucose counterparts. Moreover, it appears that the more soluble polyhydroxy fatty acid amides can help solubilize their less soluble counterparts, to varying degrees. Accordingly, the formulator may elect to use a raw material comprising a high glucose corn syrup, for example, but to select a syrup which contains a modicum of maltose (e.g., 1% or more). The resulting mixture of polyhydroxy fatty acids will, in general, exhibit more preferred solubility properties over a broader range of temperatures and concentrations than would a "pure" glucose-derived polyhydroxy fatty acid amide. Thus, in addition to any economic advantages for using sugar mixtures rather than pure sugar reactants, the polyhydroxy fatty acid amides prepared from mixed sugars can offer very substantial advantages with respect to performance and/or ease-of-formulation. Typically, the formulator electing to use such mixtures may find it advantageous to select polyhydroxy fatty acid amide mixtures which contain ratios of monosaccharides (e.g., glucose) to di- and higher saccharides (e.g., maltose) from about 4:1 to about 99:1.

The manufacture of preferred, uncyclized polyhydroxy fatty acid amides from fatty esters and N-alkyl polyols can be carried out in alcohol solvents at temperatures from about 30°C-90°C, preferably about 50°C-80°C. It has now been determined that it may be convenient for the formulator to conduct such processes in 1,2-propylene glycol solvent, since the glycol solvent need not be completely removed from the reaction product prior to use in the finished detergent formulation. The formulator may also find it convenient to run the process at 30°C-90°C in solvents which comprise ethoxylated alcohols, such as the ethoxylated (EO 3-8) C₁₂-C₁₄ alcohols, such as those available as NEODOL 23 EO6.5 (RTM) (Shell). When such ethoxylates are used, it is preferred that they not contain substantial amounts of unethoxylated alcohol and, most preferably, not contain substantial amounts of monoethoxylated alcohol.

While methods for making polyhydroxy fatty acid amides *per se* form no part of the invention herein, the formulator can also note other syntheses of polyhydroxy fatty acid amides as described hereinafter.

Typically, the industrial scale reaction sequence for preparing the preferred acyclic polyhydroxy fatty acid amides will comprise: Step 1 - preparing the N-alkyl polyhydroxy amine derivative from the desired sugar or sugar mixture by formation of an adduct of the N-alkyl amine and the sugar, followed by reaction with hydrogen in the presence of a catalyst; followed by Step 2 -reacting the aforesaid polyhydroxy amine with, preferably, a fatty ester to form an amide bond. While a variety of N-alkyl polyhydroxy amines useful in Step 2 of the reaction sequence can be prepared by various art-disclosed processes, the following process is convenient and makes use of economical sugar syrup as the raw material. It is to be understood that, for best results when using such syrup raw materials, the manufacturer should select syrups that are quite light in color or, preferably, nearly colorless ("water-white").

### Preparation of N-Alkyl Polyhydroxy Amine From Plant-Derived Sugar Syrup

I. Adduct Formation - The following is a standard process in which about 420 g of about 55% glucose solution (corn syrup - about 231 g glucose - about 1.28 moles) having a Gardner Color of less than 1 is reacted with about 119 g of about 50% aqueous methylamine (59.5 g of methylamine - 1.92 moles) solution. The methylamine (MMA) solution is purged and shielded with N₂ and cooled to about 10°C, or less. The corn syrup is purged and shielded with N₂ at a temperature of about 10°-20°C. The corn syrup is added slowly to the MMA solution at the indicated reaction temperature as shown. The Gardner Color is measured at the indicated approximate times in minutes.

**TABLE 1**

| Time in Minutes: | 10 | 30 | 60 | 120 | 180 | 240 |
|---|---|---|---|---|---|---|
| Reaction Temp. °C | Gardner Color (Approximate) | | | | | |
| 0 | 1 | 1 | 1 | 1 | 1 | 1 |
| 20 | 1 | 1 | 1 | 1 | 1 | 1 |
| 30 | 1 | 1 | 2 | 2 | 4 | 5 |
| 50 | 4 | 6 | 10 | - | - | - |

As can be seen from the above data, the Gardner Color for the adduct is much worse as the temperature is raised above about 30°C and at about 50°C, the time that the adduct has a Gardner Color below 7 is only about 30 minutes. For longer reaction, and/or holding times, the temperature should be less than about 20°C. The Gardner Color should be less than about 7, and preferably less than about 4 for good color glucamine.
When one uses lower temperatures for forming the adduct, the time to reach substantial equilibrium concentration of the adduct is shortened by the use of higher ratios of amine to sugar. With the 1.5:1 mole ratio of amine to sugar noted, equilibrium is reached in about two hours at a reaction temperature of about 30°C. At a 1.2:1 mole ratio, under the same conditions, the time is at least about three hours. For good color, the combination of amine:sugar ratio; reaction temperature; and reaction time is selected to achieve substantially equilibrium conversion, e.g., more than about 90%, preferably more than about 95%, even more preferably more than about 99%, based upon the sugar, and a color that is less than about 7, preferably less than about 4, more preferably less than about 1, for the adduct.
Using the above process at a reaction temperature of less than about 20°C and corn syrups with different Gardner Colors as indicated, the MMA adduct color (after substantial equilibrium is reached in at least about two hours) is as indicated.

**TABLE 2**

| | Gardner Color (Approximate) | | | | | | |
|---|---|---|---|---|---|---|---|
| Corn syrup | 1 | 1 | 1 | 1+ | 0 | 0 | 0+ |
| Adduct | 3 | 4/5 | 7/8 | 7/8 | 1 | 2 | 1 |

As can be seen from the above, the starting sugar material must be very near colorless in order to consistently have adduct that is acceptable. When the sugar has a Gardner Color of about 1, the adduct is sometimes acceptable and sometimes not acceptable. When the Gardner Color is above 1 the resulting adduct is unacceptable. The better the initial color of the sugar, the better is the color of the adduct.
II. Hydrogen Reaction - Adduct from the above having a Gardner Color of 1 or less is hydrogenated according to the following procedure.
About 539 g of adduct in water and about 23.1 g of United Catalyst G49B Ni catalyst are added to a one liter autoclave and purged two times with 1378.8 kPa (200 psig) H₂ at about 20°C. The H₂ pressure is raised to about 9651.6 kPa (1400 psi) and the temperature is raised to about 50°C. The pressure is then raised to about 11030.4 kPa (1600 psig) and the temperature is held at about 50-55°C for about three hours. The product is about 95% hydrogenated at this point. The temperature is then raised to about 85°C for about 30 minutes and the reaction mixture is decanted and the catalyst is filtered out. The product, after removal of water and MMA by evaporation, is about 95% N-methyl glucamine, a white powder.

The above procedure is repeated with about 23.1 g of Raney Ni catalyst with the following changes. The catalyst is washed three times and the reactor, with the catalyst in the reactor, is purged twice with 1378.8 kPa (200 psig) H₂ and the reactor is pressurized with H₂ at 11030.4 kPa (1600 psig) for two hours, the pressure Is released at one hour and the reactor is repressurized to 11030.4 kPa (1600 psig). The adduct is then pumped into the reactor which is at 1378.8 kPa (200 psig) and 20°C, and the reactor is purged with 1378.8 kPa (200 psig) H₂, etc., as above.

The resulting product in each case is greater than about 95% N-methyl glucamine; has less than about 10 ppm Ni based upon the glucamine; and has a solution color of less than about Gardner 2.

The crude N-methyl glucamine is color stable to about 140°C for a short exposure time.

It is important to have good adduct that has low sugar content (less than about 5%, preferably less than about 1%) and a good color (less than about 7, preferably less than about 4 Gardner, more preferably less than about 1).

In another reaction, adduct is prepared starting with about 159 g of about 50% methylamine in water, which is purged and shielded with N₂ at about 10-20°C. About 330 g of about 70% corn syrup (near water-white) is degassed with N₂ at about 50°C and is added slowly to the methylamine solution at a temperature of less than about 20°C. The solution is mixed for about 30 minutes to give about 95% adduct that is a very light yellow solution.

About 190 g of adduct in water and about 9 g of United Catalyst G49B Ni catalyst are added to a 200 ml autoclave and purged three times with H₂ at about 20°C. The H₂ pressure is raised to about 1378.8 kPa (200 psi) and the temperature is raised to about 50°C. The pressure is raised to 1723.5 kPa (250 psi) and the temperature is held at about 50-55°C for about three hours. The product, which is about 95% hydrogenated at this point, is then raised to a temperature of about 85°C for about 30 minutes and the product, after removal of water and evaporation, is about 95% N-methyl glucamine, a white powder.

It is also important to minimize contact between adduct and catalyst when the H₂ pressure is less than about 6894 kPa (1000 psig) to minimize Ni content in the glucamine. The nickel content in the N-methyl glucamine in this reaction is about 100 ppm as compared to the less than 10 ppm in the previous reaction.

The following reactions with H₂ are run for direct comparison of reaction temperature effects.

A 200 ml autoclave reactor is used following typical procedures similar to those set forth above to make adduct and to run the hydrogen reaction at various temperatures.

Adduct for use in making glucamine is prepared by combining about 420 g of about 55% glucose (corn syrup) solution (231 g glucose; 1.28 moles) (the solution is made using 99DE (RTM) corn syrup from CarGill, the solution having a color less than Gardner 1) and about 119 g of 50% methylamine (59.5 g MMA; 1.92 moles) (from Air Products).

The reaction procedure is as follows:
1. Add about 119 g of the 50% methylamine solution to a N₂ purged reactor, shield with N₂ and cool down to less than about 10°C.
2. Degas and/or purge the 55% corn syrup solution at 10-20°C with N₂ to remove oxygen in the solution.
3. Slowly add the corn syrup solution to the methylamine solution and keep the temperature less than about 20°C.
4. Once all corn syrup solution is added in, agitate for about 1-2 hours.

The adduct is used for the hydrogen reaction right after making, or is stored at low temperature to prevent further degradation.

The glucamine adduct hydrogen reactions are as follows:
1. Add about 134 g adduct (color less than about Gardner 1) and about 5.8 g G49B Ni to a 200 ml autoclave.
2. Purge the reaction mix with about 1378.8 kPa (200 psi) H₂ twice at about 20-30°C.
3. Pressure with H₂ to about 2757.6 kPa (400 psi) and raise the temperature to about 50°C.
4. Raise pressure to about 3447 kPa (500 psi), react for about 3 hours. Keep temperature at about 50-55°C. Take Sample 1.
5. Raise temperature to about 85°C for about 30 minutes.
6. Decant and filter out the Ni catalyst. Take Sample 2.

Conditions for constant temperature reactions:
1. Add about 134 g adduct and about 5.8 g G49B Ni to a 200 ml autoclave.
2. Purge with about 1378.8 kPa (200 psi) H₂ twice at low temperature.
3. Pressure with H₂ to about 2757.6 kPa (400 psi) and raise temperature to about 50°C.
4. Raise pressure to about 3447 kPa (500 psi), react for about 3.5 hours. Keep temperature at indicated temperature.
5. Decant and filter out the Ni catalyst. Sample 3 is for about 50-55°C; Sample 4 is for about 75°C; and Sample 5 is for about 85°C. (The reaction time for about 85°C is about 45 minutes.)

All runs give similar purity of N-methyl glucamine (about 94%); the Gardner Colors of the runs are similar right after reaction, but only the two-stage heat treatment gives good color stability; and the 85°C run gives marginal color immediately after reaction.

### EXAMPLE VI

The preparation of the tallow (hardened) fatty acid amide of N-methyl maltamine for use in compositions according to this invention is as follows.
Step 1 - Reactants: Maltose monohydrate (Aldrich, lot 01318KW); methylamine (40 wt% in water) (Aldrich, lot 03325TM); Raney nickel, 50% slurry (UAD 52-73D, Aldrich, lot 12921LW).
   The reactants are added to glass liner (250 g maltose, 428 g methylamine solution, 100 g catalyst slurry - 50 g Raney Ni) and placed in 3 L rocking autoclave, which is purged with nitrogen 3 x 3447 kPa (3X500 psig) and hydrogen 2 x 3447 kPa (2X500 psig) and rocked under H₂ at room temperature over a weekend at temperatures ranging from 28°C to 50°C. The crude reaction mixture is vacuum filtered 2X through a glass microfiber filter with a silica gel plug. The filtrate is concentrated to a viscous material. The final traces of water are azetroped off by dissolving the material in methanol and then removing the methanol/water on a rotary evaporator. Final drying is done under high vacuum. The crude product is dissolved in refluxing methanol, filtered, cooled to recrystallize, filtered and the filter cake is dried under vacuum at 35°C. This is cut #1. The filtrate is concentrated until a precipitate begins to form and is stored in a refrigerator overnight. The solid is filtered and dried under vacuum. This is cut #2. The filtrate is again concentrated to half its volume and a recrystallization is performed. Very little precipitate forms. A small quantity of ethanol is added and the solution is left in the freezer over a weekend. The solid material is filtered and dried under vacuum. The combined solids comprise N-methyl maltamine which is used in Step 2 of the overall synthesis.
Step 2 - Reactants: N-methyl maltamine (from Step 1); hardened tallow methyl esters; sodium methoxide (25% in methanol); absolute methanol (solvent); mole ratio 1:1 amine:ester; initial catalyst level 10 mole % (w/r maltamine), raised to 20 mole %; solvent level 50% (wt.).
   In a sealed bottle, 20.36 g of the tallow methyl ester is heated to its melting point (water bath) and loaded into a 250 ml 3-neck round-bottom flask with mechanical stirring. The flask is heated to *ca*. 70°C to prevent the ester from solidifying. Separately, 25.0 g of N-methyl maltamine is combined with 45.36 g of methanol, and the resulting slurry is added to the tallow ester with good mixing. 1.51 g of 25% sodium methoxide in methanol is added. After four hours the reaction mixture has not clarified, so an additional 10 mole % of catalyst (to a total of 20 mole %) is added and the reaction is allowed to continue overnight (*ca.* 68°C) after which time the mixture is clear. The reaction flask is then modified for distillation. The temperature is increased to 110°C. Distillation at atmospheric pressure is continued for 60 minutes. High vacuum distillation is then begun and continued for 14 minutes, at which time the product is very thick. The product is allowed to remain in the reaction flask at 110°C (external temperature) for 60 minutes. The product is scraped from the flask and triturated in ethyl ether over a weekend. Ether is removed on a rotary evaporator and the product is stored in an oven overnight, and ground to a powder. Any remaining N-methyl maltamine is removed from the product using silica gel. A silica gel slurry in 100% methanol is loaded into a funnel and washed several times with 100% methanol. A concentrated sample of the product (20 g in 100 ml of 100% methanol) is loaded onto the silica gel and eluted several times using vacuum and several methanol washes. The collected eluant is evaporated to dryness (rotary evaporator). Any remaining tallow ester is removed by trituration in ethyl acetate overnight, followed by filtration. The filter cake is then vacuum dried overnight. The product is the tallowalkyl N-methyl maltamide.

In an alternate mode, Step 1 of the foregoing reaction sequence can be conducted using commercial corn syrup comprising glucose or mixtures of glucose and, typically, 5%, or higher, maltose. The resulting polyhydroxy fatty acid amides and mixtures can be used in any of the compositions herein.

In still another mode, Step 2 of the foregoing reaction sequence can be carried out in 1,2-propylene glycol or NEODOL (RTM). At the discretion of the formulator, the propylene glycol or NEODOL (RTM) need not be removed from the reaction product prior to its use to formulate shampoo compositions. Again, according to the desires of the formulator, the methoxide catalyst can be neutralized by citric acid to provide sodium citrate, which can remain in the polyhydroxy fatty acid amide.

### EXAMPLE VII

In any of the foregoing examples, the fatty acid glucamide surfactant can be replaced by an equivalent amount of the maltamide surfactant, or mixtures of glucamide/maltamide surfactants derived from plant sources. In the compositions the use of ethanolamides appears to help cold temperature stability of the finished formulations. Moreover, the use of sulfobetaine (aka "sultaine") surfactants provides superior sudsing.

For compositions where especially high sudsing compositions are desired, it is preferred that less than 5%, more preferably less than 2% by weight, most preferably substantially no C₁₄ or higher fatty acids be present, since these can suppress sudsing. Accordingly, the formulator of high sudsing compositions will desirably avoid the introduction of suds-suppressing amounts of such fatty acids into high sudsing compositions with the polyhydroxy fatty acid amides, and/or avoid the formation of C₁₄ and higher fatty acids on storage of the finished compositions. One simple means is to use C₁₂ ester reactants to prepare the polyhydroxy fatty acid amides herein. Fortunately, the use of sultaine (or, less preferably for shampoos) amine oxide surfactants can overcome some of the negative sudsing effects caused by the fatty acids.

It will be appreciated by those skilled in the chemical arts that the preparation of the polyhydroxy fatty acid amides herein using the di- and higher saccharides such as maltose will result in the formation of polyhydroxy fatty acid amides wherein linear substituent Z is "capped" by a polyhydroxy ring structure.

## Claims

1. A shampoo composition comprising a silicone-containing hair conditioning agent at a level of from 0,05%-10% by weight; said composition optionally comprising one or more adjunct surfactants, optional thickening agents and fluid carrier, said composition being characterized in that it comprises at least 1%, preferably at least 3%, by weight of a polyhydroxy fatty acid amide surfactant.

2. A composition according to Claim 1 wherein said polyhydroxy fatty acid amide surfactant is of the formula R²C(O)NR¹CH₂-(CH₂OH)₄CH₂OH, wherein R² is C₁₁-C₁₇ alkyl and R¹ is methyl.

3. A composition according to Claim 1 wherein said polyhydroxy fatty acid amide is derived from maltose.

4. A composition according to Claim 1 wherein said polyhydroxy fatty acid amide is derived from a mixture of monosaccharides, disaccharides and, optionally, higher saccharides, said mixture comprising at least 1% by weight of at least one disaccharide, preferably maltose.

5. A composition according to Claim 1 wherein the silicone containing hair conditioning agent is used in conjunction with a thickening or suspending agent, preferably ethylene glycol distearate.

6. A composition according to Claim 1 which additionally contains a suds-enhancing amount of an alkylaminoalkanoate or alkyliminodialkanoate surfactant, preferably from 1% to 10% by weight of lauroiminodipropionate.

7. A method for enhancing the deposition onto hair of a silicone-containing hair conditioning agent at a level of from 0,05%-10% by weight; from a surfactant-containing shampoo vehicle, characterized in that said shampoo vehicle contains at least 1%, preferably at least 3%, by weight of a polyhydroxy fatty acid amide, preferably a C₁₁-C₁₇ N-methyl glucamide, C₁₁-C₁₇ N-methyl meltamide, or mixtures of said glucamide and maltamide, and said shampoo vehicle preferably also containing from 1% to 10% by weight of an alkylaminoalkanoate or alkyliminodialkanoate, most preferably lauroiminodipropionate, and shampooing the hair with said shampoo vehicle.

## Patentansprüche

1. Shampoozusammensetzung, umfassend ein Silicon enthaltendes Haarkonditionierungsmittel in einer Menge von 0,05 Gew.-% bis 10 Gew.-%, welche Zusammensetzung wahlweise ein oder mehrere zusätzliche grenzflächenaktive Mittel, fakultative Verdickungsmittel und einen flüssigen Träger enthält, wobei die Zusammensetzung dadurch gekennzeichnet ist, daß sie mindestens 1 Gew.-%, vorzugsweise mindestens 3 Gew.-% von einem Polyhydroxyfettsäureamid-grenzflächenaktiven Mittel enthält.

2. Zusammensetzung nach Anspruch 1, wobei das genannte Polyhydroxyfettsäureamid-grenzflächenaktive Mittel die Formel R²C(O)NR¹CH₂(CH₂OH)₄CH₂OH besitzt, worin R² für C₁₁-C₁₇-Alkyl steht und R¹ Methyl ist.

3. Zusammensetzung nach Anspruch 1, wobei sich das genannte Polyhydroxyfettsäureamid aus Maltose herleitet.

4. Zusammensetzung nach Anspruch 1, wobei sich das genannte Polyhydroxyfettsäureamid von einem Gemisch aus Monosacchariden, Disacchariden und, wahlweise, höheren Sacchariden herleitet, welches Gemisch mindestens 1 Gew.-% von mindestens einem Disaccharid, vorzugsweise Maltose, enthält.

5. Zusammensetzung nach Anspruch 1, worin das Silicon enthaltende Haarkonditionierungsmittel in Verbindung mit einem Verdickungs- oder Suspendierungsmittel, vorzugsweise Ethylenglykoldistearat, verwendet wird.

6. Zusammensetzung nach Anspruch 1, welche zusätzlich eine den Schaum erhöhende Menge von einem Alkylaminoalkanoat- oder einem Alkyliminodialkanoat-grenzflächenaktiven Mittel, vorzugsweise 1 Gew.-% bis 10 Gew.-% an Lauroiminodipropionat, enthält.

7. Verfahren zur Erhöhung der Ablagerung von einem ein Silicon enthaltenden Haarkonditionierungsmittel in einer Menge von 0,05 Gew.-% bis 10 Gew.-% aus einem ein grenzflächenaktives Mittel enthaltenden Shampoo-Träger auf Haar, dadurch gekennzeichnet, daß der genannte Shampoo-Träger mindestens 1 Gew.-%, vorzugsweise mindestens 3 Gew.-% von einem Polyhydroxyfettsäureamid, vorzugsweise einem C₁₁-C₁₇-N-Methylglucamid, C₁₁-C₁₇-N-Methylmaltamid oder von Gemischen aus dem Glucamid und dem Maltamid enthält, und daß der genannte Shampoo-Träger vorzugsweise auch 1 Gew.-% bis 10 Gew.-% von einem Alkylaminoalkanoat oder einem Alkyliminodialkanoat, am stärksten bevorzugt Lauroiminodipropionat, enthält, und das Haar mit dem genannten Shampoo-Träger shampooniert wird.

## Revendications

1. Composition de shampooing comprenant un agent de conditionnement des cheveux contenant de la silicone à un niveau de 0,05 à 10 % en poids, ladite composition comprenant en option un ou plusieurs agents tensioactifs auxiliaires, des agents épaississants facultatifs et un véhicule fluide, ladite composition étant caractérisée en ce qu'elle comprend au moins 1 %, de préférence au moins 3 %, en poids d'un agent tensioactif d'amide d'acide gras polyhydroxylé.

2. Composition selon la revendication 1, dans laquelle ledit agent tensioactif d'amide d'acide gras polyhydroxylé a pour formule R²C(O)NR¹CH₂-(CH₂OH)₄CH₂OH, dans laquelle R² est un groupe alkyle en C₁₁-C₁₇ et R¹ est un groupe méthyle.

3. Composition selon la revendication 1, dans laquelle ledit amide d'acide gras polyhydroxylé est dérivé du maltose.

4. Composition selon la revendication 1, dans laquelle ledit amide d'acide gras polyhydroxylé est dérivé d'un mélange de monosaccharides, de disaccharides et, en option, de saccharides supérieurs, ledit mélange comprenant au moins 1 % en poids d'au moins un disaccharide, de préférence le maltose.

5. Composition selon la revendication 1, dans laquelle l'agent de conditionement des cheveux contenant de la silicone est utilisé conjointement avec un agent d'épaississement ou de suspension, de préférence le distéarate d'éthylène glycol.

6. Composition selon la revendication 1, contenant en outre une quantité promotrice de mousse d'un agent tensioactif de type alkylaminoalcanoate ou alkyliminodialcanoate, de préférence 1 à 10 % en poids de lauroiminodipropionate.

7. Procédé permettant de favoriser le dépôt sur les cheveux d'un agent de conditionnement des cheveux contenant de la silicone à un niveau de 0,05 à 10 % en poids à partir d'un véhicule de shampooing contenant un agent tensioactif, caractérisé en ce que ledit véhicule de shampooing contient au moins 1 %, de préférence au moins 3 %, en poids d'un amide d'acide gras polyhydroxylé, de préférence un N-méthyl glucamide en C₁₁-C₁₇, un N-méthyl maltamide en C₁₁-C₁₇ ou des mélanges desdits glucamide et maltamide, ledit véhicule de shampooing contenant également de préférence 1 à 10 % en poids d'un alkylaminoalcanoate ou d'un alkyliminodialcanoate, mieux encore du lauroiminodipropionate, ledit procédé consistant à appliquer sur les cheveux ledit véhicule de shampooing.
